# EUROPEAN PATENT SPECIFICATION

(11) **EP 3 294 317 B1**
(45) Date of publication and mention of the grant of the patent: **09.12.2020**
(21) Application number: 16723338.6
(22) Date of filing: 12.05.2016
(51) Int. Cl.: A61K 31/17, A61K 38/12, A61K 45/06, A61K 31/407, A61K 31/454, A61K 31/496, A61K 31/505, A61K 31/5383, A61K 31/635, A61K 31/7036, A61K 31/65, A61P 31/04, A61K 47/10, A61K 47/20

(54) **ENHANCED ANTIBIOTIC COMPOSITION**
VERBESSERTE ANTIBIOTISCHE ZUSAMMENSETZUNG
COMPOSITION ANTIBIOTIQUE AMÉLIORÉE

(30) Priority: 14.05.2015 EP 15167740
(43) Date of publication of application: 21.03.2018
(73) Proprietor: Universitat Autònoma de Barcelona, 08193 Bellaterra (Cerdanyola del Valles) (ES); Fundació Institució Catalana De Recerca I Estudis Avançats, 08010 Barcelona (ES)
(72) Inventor: GIBERT GONZALEZ, Isidre, 08193 Bellaterra (Cerdanyola del Vallès) (ES); DAURA RIBERA, Xavier, 08193 Bellaterra (Cerdanyola del Vallès) (ES); YERO CORONA, Daniel, 08193 Bellaterra (Cerdanyola del Vallès) (ES); MARTIN, Indarte, 08193 Bellaterra (Cerdanyola del Vallès) (ES); CONCHILLO SOLÉ, Oscar, 08193 Bellaterra (Cerdanyola del Vallès) (ES)
(74) Representative: Ponti & Partners, S.L.P
(86) International application number: PCT/EP2016/060664
(87) International publication number: WO 2016/180919

(56) References cited:
- WO-A2-2008/076806
- Anonymous: "SID 130457895 - PubChem T6840188", , 6 December 2011 (2011-12-06), XP055286017, Retrieved from the Internet: URL:https://pubchem.ncbi.nlm.nih.gov/subst ance/130457895#section=Top [retrieved on 2016-07-05]
- PATRICIA BERNAL ET AL: "Antibiotic adjuvants: identification and clinical use", MICROBIAL BIOTECHNOLOGY, vol. 6, no. 5, 28 February 2013 (2013-02-28), pages 445-449, XP055286088, GB ISSN: 1751-7915, DOI: 10.1111/1751-7915.12044
- PATRICIA L. TAYLOR ET AL: "A Forward Chemical Screen Identifies Antibiotic Adjuvants in Escherichia coli", ACS CHEMICAL BIOLOGY, vol. 7, no. 9, 21 September 2012 (2012-09-21), pages 1547-1555, XP055286027, US ISSN: 1554-8929, DOI: 10.1021/cb300269g

## Description

### Technical Field

The present invention relates to the use of potentiator compounds for enhancing the antibacterial activity of known antibiotics in front of multidrug resistant bacteria.

### Technical Background

Progress in the antibiotic treatment of bacterial infections has considerably reduced mortality from many infectious diseases.

However, the continuous use of antimicrobial agents increases selection pressure favouring the emergence, multiplication, and spread of resistant strains. The rate of antimicrobial resistance among nosocomial pathogens is increasing for nearly all antimicrobial-pathogen combinations that have been examined. A nosocomial pathogen is a pathogen acquired in hospitals and other healthcare facilities.

Therapy is becoming ever more difficult because of the increasing rate of antimicrobial resistance among common nosocomial pathogens. Over the last decade, multidrug-resistant Gram-negative bacteria (MDR-GNB), including MDR-*Pseudomonas aeruginosa, MDR-Acinetobacter baumannii* and *Enterobacteriaceae* producing extended-spectrum β-lactamases (ESBL) and carbapenemases, have been implicated in severe hospital acquired infections and their occurrence has increased steadily. The World Health Organization (WHO) has recently identified antimicrobial resistance as one of the three most important problems facing human health.

The pathogens associated with hospital-acquired infections are similar worldwide. Many different bacteria, viruses, fungi and parasites may cause nosocomial infections. Virtually every pathogen has the potential to cause infection in hospitalized patients but only a limited number of both Gram-positive and Gram-negative bacteria are responsible for the majority of nosocomial infections. Gram-positive bacteria are the commonest cause of nosocomial infections with *Staphylococcus aureus* being the predominant pathogen. However, overall trends for Gram-negative pathogens in ICUs showed significant increases during the last decades. S. *aureus* is usually found in infections due to gram negative bacteria being an opportunistic infection. Therefore, antibiotics with a broader spectrum of coverage - particularly those with activity against multidrug-resistant Gram-negative bacilli, and drug-resistant *S*. *aureus* - should be considered for patients who have risk factors for opportunistic infections

Gram-negative bacteria are a class of bacteria that do not retain the crystal violet stain used in the Gram staining method of bacterial differentiation. Many of these bacteria can be cause of pneumonia, bloodstream infections, wound or surgical site infections, and meningitis in healthcare settings. Additionally, these organisms are highly efficient at up-regulating or acquiring genes that code for mechanisms of antibiotic drug resistance, especially in the presence of antibiotic selection pressure. Therefore, many Gram-negative bacteria are resistant to multiple drugs and are increasingly resistant to most available antibiotic classes. Among the Gram-negative bacteria responsible for the majority of nosocomial infections there are *Escherichia coli, Pseudomonas aeruginosa, Acinetobacter baumanni, Klebsiella pneumoniae, Serratia marcescens, Stenotrophomonas maltophilia, Enterobacter spp.* and *Salmonella spp.*

Antimicrobial resistance is a natural response of bacteria to antibiotic exposure and can be intrinsic to a bacterium, arise from spontaneous genetic mutations, or be associated with horizontal gene transfer. There are a variety of mechanisms of resistance to currently used antimicrobial agents in MDR-GNB. In very broad terms, these mechanisms can either chemically modify the antibiotic to render it inactive, prevent its uptake (altered membrane permeability), pump it out of the cell (efflux pumps), or modify target site so that it is not recognized by the antibiotic. Bacteria can also adapt to the presence of toxic levels of antibiotics using several types of responses, including regulatory mechanisms that alter cellular physiology or genetic changes. During adaptive resistance, a bacterium might upregulate appropriate resistance genes when exposed to an antibiotic, which is followed by down-regulation of the gene when this pressure is removed.

In the prior art several antibiotic therapies for nosocomial pathogens have been disclosed, which are based mainly on combinations of drugs. With the increase of antibiotic resistance, there is a higher possibility of adequate antibacterial coverage by combining two antibacterial agents than using a single agent. Antibiotic combination therapy permits to explore different molecular targets of individual agents and thereby broaden the spectrum of action. Rational antibiotic combination therapy decreases the concentration/dose required for treatment and thus reduces the dose-related toxicity. Antibacterial agents with broad spectra of activity and multimodal action may prevent emergence of drug resistance when acting in combination. Synergistic action leading to broader spectrum than the sum of activities of two individual agents has been reported with combination therapy.

In Lynch, T. J., Choosing optimal antimicrobial therapies, Med. Clin. North Am., 2012, 96, 1079-1094, it is disclosed the use of antipseudomonal β-lactams such as cefepime, ceftazidime, piperacillin-tazobactam or doripenem, as well as the fluoroquinolone ciprofloxacin for the treatment of nosocomial infections by *P. aeruginosa* and other highly resistant organisms.

In Farrell et al., Antimicrobial susceptibilities of a worldwide collection of Stenotrophomonas maltophilia isolates tested against tigecycline and agents commonly used for S. maltophilia infections, Antimicrob. Agents Chemother., 2010, 54, 2735-2737, it is disclosed the combination trimethoprim-sulfamethoxazole (TMP/SMX) for the treatment of infections caused by *S*. *maltophilia.*

In Jean et al., Current review of antimicrobial treatment of nosocomial pneumonia caused by multidrug-resistant pathogens, Expert Opin. Pharmacother., 2011, 12, 2145-2148, it is recommended the use of a carbapenem (imipenem or meropenem) plus sulbactam or colistin or tigecycline in combination with other agents (imipenem, amikacin or rifampin) for the treatment of nosocomial pneumonia due to drug-resistant *A. baumannii.*

In Tamma et al., Combination therapy for treatment of infections with gram-negative bacteria, Clin. Microbiol. Rev., 2012, 25, 450-470, it is disclosed the combination of ampicillin and gentamicin in Enterococcal endocarditis. However, it is also disclosed that there are certain disadvantages of antibiotic combination therapy, which eventually can generate antagonism between the drugs.

Other risks associated with combination therapy are fungal overgrowth, drug-drug interactions, drug toxicity, irrational drug use and increase in cost of therapy, as disclosed in Mehta et al., Burden of Antibiotic Resistance in Common Infectious Diseases: Role of Antibiotic Combination Therapy, J. Clin. Diagn. Res. JCDR, 2014, 8, ME05-ME08.

The appearance of new multiresistant pathogens, such as imipenem-resistant *P. aeruginosa* and/or *A. baumannii,* carbapenem-resistant Gram-negative bacteria harbouring carbapenemases, and vancomycin-resistant *Enterococcus spp.,* has determined the use of new antibacterials, the reintroduction of drugs that were removed in the past due to toxicity or the use of combinations with *in vitro* synergy. In particular, the pharmaceutical pipeline of antibiotics active against MDR-GNB is very limited. New antibiotics that have been discovered and introduced into clinical practice in the last few years are active mostly against Gram-positive organisms, whereas when targeting resistant Gram-negative bacteria, clinicians are forced to rediscover old drugs, such as polymyxins and fosfomycin.

Among new antibacterials active against Gram-negative microorganisms that are already in the market, tigecycline and doripenem, seem the most promising. Tigecycline belongs to the novel glycylcycline class of antibiotics, which was reported to be the most effective antibiotic in front of 1,586 S. *maltophilia* clinical isolates. However, decreased susceptibility to tigecycline has been already reported, and *P. aeruginosa* displays high levels of resistance to tigecycline. On the other hand, doripenem offers only modest improvements to an existing class of drugs and its use is hindered by high levels of pre-existing carbapenem resistance.

A less expensive approach is the combination of non-antibiotic compounds with already existing antibiotics. An alternative to the development of new antibiotics is to find potentiators or adjuvants (as disclosed in Bernal et al., Antibiotic adjuvants: identification and clinical use, Microb. Biotechnol., 2013, 6, 445-449) of the already existing ones. The most successful and clinically used strategy to date has been the combination of a β-iactam antibiotic with clavulanic acid, a β-iactamase inhibitor adjuvant. To increase the antibacterial activity of antibiotics, potentiators may have different possibilities, for example, enhancing the antibiotic entrance into cells, preventing antibiotics from being pumped out the bacterial cells, or inhibiting regulatory mechanisms that control bacterial virulence.

In Kubo et al., Indole and (E)-2-hexenal, phytochemical potentiators of polymyxins against Pseudomonas aeruginosa and Escherichia coli. Antimicrob. Agents Chemother., 1996, 40, 1438-1441, it is disclosed that various degrees of potentiation may be obtained by combining polymyxins and phytochemicals. In this case both phytochemicals have broad spectra of antimicrobial activity, but later it was found that indole enhances the antimicrobial resistance of *P. aeruginosa* (Lee et al., Indole and 7-hydroxyindole diminish P. aeruginosa virulence, Microb. Biotechnol., 2009, 2, 75-90) and *E. coli* (Lee et al., Bacterial charity work leads to population-wide resistance, Nature, 2010, 467, 82-85). WO2008/076806 discloses a composition comprising antibiotics and enhancers of antibiotics such as mefloquine.

There is, thus, a need to provide new antibiotic compositions showing enhanced antibacterial activity against multiresistant bacteria.

### Object of the invention

The object of the present invention is an antibiotic composition.

It forms also part of the object of the present invention the antibiotic composition for use as a medicament.

It forms also part of the object of the present invention a potentiator compound for use as enhancer of the antibacterial activity of antibiotics.

### Detailed description of the invention

It is the object of the present invention an antibiotic composition, which comprises a therapeutic amount of an antibiotic, one potentiator compound which is a compound of general formula (I): 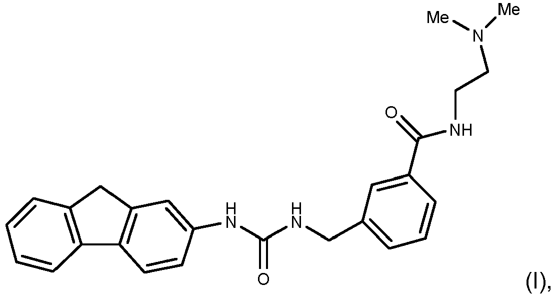 and at least one pharmaceutically acceptable excipient.

It is also disclosed herein an antibiotic composition, which comprises a therapeutic amount of an antibiotic, one or more potentiator compounds selected from the group consisting of:
a) a compound of general formula (I): 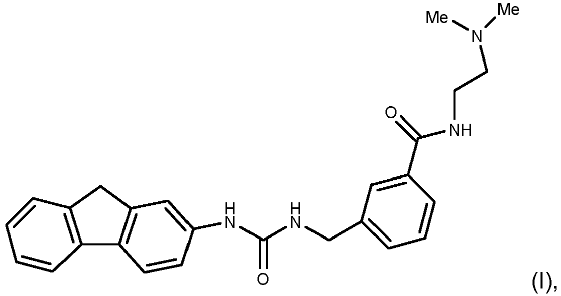 and
b) a the compound of formula (II): 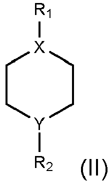 wherein:
   when X = Y = N
   R₁ is 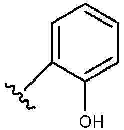
   and R₂ is 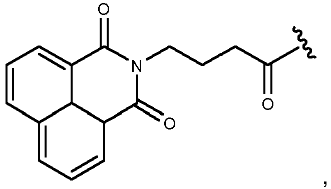
   or R₁ is 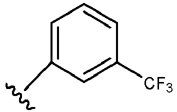
   and R₂ is 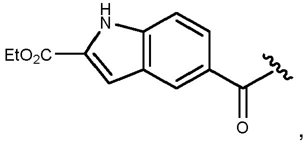
   or R₁ is 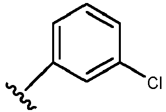
   and R₂ is 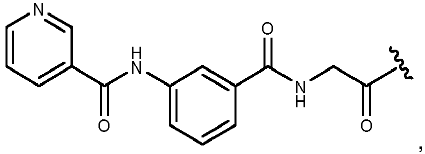 and
   when X = CH and Y = N,
   R₁ is 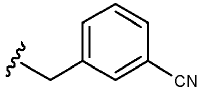
   and R₂ is 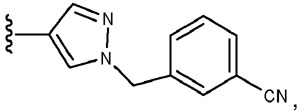 and at least one pharmaceutically acceptable excipient.

The authors of the present invention have identified several compounds, which surprisingly increase the susceptibility to antibiotics in front of the most common agents (Gram-negative bacteria) responsible of nosocomial infections and in front of several resistant clinical isolates. The combination of colistin, a polymyxin antibiotic, or levofloxacin, a fluoroquinolone antibiotic, or tigecycline, a glycylcycline antibiotic (tetracycline analogue), or imipenem, a carbapenem antibiotic, or ciprofloxacin, a quinolone antibiotic, or amikacin, an aminoglycoside antibiotic, for example, with such compounds surprisingly enhances the effectivity of the corresponding antibiotic in front of several nosocomial pathogenic strains. By enhancing the activity of the antibiotic with potentiator compounds, it may be reduced the amount of antibiotic used, thus lowering its toxicity, or the amount of antibiotic can be maintained, thus delivering a more potent medicine.

In the context of the present invention, a potentiator is an adjuvant that increases the activity of an antibiotic in front of specific bacteria. For each bacterium, this increase in the activity of the antibiotic is calculated by dividing the minimal inhibitory concentration of the antibiotic without adjuvant, by the minimal inhibitory concentration of the antibiotic with the adjuvant. In other words, in the presence of an adjuvant, it is necessary a lower concentration of antibiotic to inhibit the growth of bacteria.

In the present description as well as in the claims, the singular forms "a" and "an" include also the plural reference unless the context clearly indicates otherwise.

### Antibiotic

The antibiotic component of the composition of the present invention is usually selected from aminoglycosides like, for example, amikacin, gentamicin, kanamycin, neomycin, metilmicin, tobramycin, and monomycin; carbapenems like, for example, ertapenem, doripenem, imipenem or meropenem; cephalosporins like, for example, cefoperazone (third-generation), ceftazidime (third-generation), ceftriaxone (third-generation), cefepime (fourth-generation), cefpirome (fourth-generation), or ceftobiprole (fifth-generation); macrolides like, for example, azithromycin, clarithromycin, or erythromycin; monobactams like, for example, aztreonam; penicillins like, for example, amoxicillin, optionally combined with clavulanate, azlocillin, ampicillin, optionally combined with sulbactam, piperacillin, optionally combined with tazobactam, or ticarcillin, optionally combined with clavulanate; polymyxins like, for example, colistin, or polymyxin B; quinolones like, for example, nalidixic acid; fluoroquinolones like, for example, ciprofloxacin, enoxacin, gatifloxacin, gemifloxacin, levofloxacin, moxifloxacin, or norfloxacin; sulphonamides like, for example, mafenide, sulfadiazine, sulfadimethoxine, sulfisoxazole, or sulfamethoxazole, optionally combined with trimethoprim; tetracyclines like, for example, doxycycline, minocycline, oxytetracycline, tigecycline, or tetracycline; chloramphenicol and fosfomycin.

Aminoglycosides display bactericidal activity and are effective mainly against Gram-negative aerobic bacteria such as *Escherichia coli* and *Klebsiella sp.* particularly *Pseudomonas aeruginosa.*

Carbapenems are a class of β-Lactam antibiotics with a broad spectrum of antibacterial activity. They are bactericidal for both Gram-positive and Gram-negative organisms and therefore useful for empiric broad-spectrum antibacterial coverage.

Cephalosporins are bactericidal agents and are structurally and pharmacologically related to penicillin. The third generation cephalosporins have a marked activity against gram-negative bacteria due to enhanced beta-lactamase stability and the ability to penetrate the gram-negative cell wall. Particularly, they are active against *P. seudomonas aeruginosa.* Cefepime and cefpirome are highly active against nosocomial pathogens. Ceftobiprole is a very broad-spectrum cephalosporin with activity against gram-positive cocci, and many Gram-negative bacilli including AmpC producing *E. coli* and *P. aeruginosa.*

Macrolides are bacteriostatic with a broad spectrum of activity against many Gram-positive bacteria and some Gram-negative bacteria. They are used in the treatment of upper and lower respiratory tract infections.

Penicillins, quinolones, fluoroquinolones, sulphonamides and tetracyclines are used in the treatment of a wide range of infections.

In a preferred embodiment, the antibiotic is selected from aminoglycosides like, for example, amikacin, gentamicin, or tobramycin; carbapenems like, for example, imipenem or meropenem; cephalosporins like, for example, ceftazidime, cefepime, or ceftriaxone; penicillins like, for example, piperacillin, optionally combined with tazobactam; polymyxins like, for example, colistin, or polymyxin B; quinolones like, for example, levofloxacin; sulphonamides like, for example, sulfamethoxazole, optionally combined with trimethoprim; and tetracyclines like, for example, minocycline, or tigecycline.

In a preferred embodiment the antibiotic is selected from the group consisting of polymyxin antibiotics, ciprofloxacin, amikacin, levofloxacin, tigecycline, imipenem and the combination trimethoprim/sulfamethoxasole, preferably in a relationship of 1:5 or 1:19.

In a more preferred embodiment the antibiotic is selected from polymyxin antibiotics.

Polymyxin antibiotics belong to a family of natural antimicrobial cationic lipopeptides, which were discovered during the mid-20^{th} century. They are used as a last resort treatment for life threatening severe systemic infections caused by MDR-Gram-negative bacteria.

Two members of the polymyxin group of antimicrobial agents are in clinical use: polymyxin B and polymyxin E, which is also termed colistin. The main difference between the molecules of colistin and polymyxin B is that the latter contains phenylalanine. Both polymyxin B and colistin are mixtures of structurally related compounds that differ from each other in the chemical structure of their fatty acid.

Colistin consists of a cationic cyclic decapeptide linked to a fatty acid chain through an α-amide linkage (molecular weight 1750 Da). The amino acid components in the molecule of colistin are D-leucine, L-threonine, and L-α-γ-diaminobutyric acid.

Colistin is active against most gram-negative aerobic bacilli. Among the most common nosocomial Gram-negative bacteria, colistin has excellent bactericidal activity against *Acinetobacter* species, *P. aeruginosa, Klebsiella* species, *Enterobacter* species, *E. coli, S. maltophilia* and *Salmonella* species.

Colistin can be also administered as a prodrug in form of colistimethate sodium, which is readily hydrolized to form sulfomethylated derivatives, as well as colistin sulfate, the active form of the drug.

In a preferred embodiment, the polymyxin antibiotic is selected from colistin, colistimethate, and pharmaceutically acceptable salts thereof, in a more preferred embodiment the polymyxin antibiotic is selected from colistin, colistin sulfate and colistimethate sodium, in a yet more preferred embodiment is colistin or colistin sulfate.

Ciprofloxacin, amikacin, levofloxacin, tigecycline, imipenem, trimethoprim and sulfamethoxasole are available commercially, for example, through the company Sigma-Aldrich (www.sigmacldrich.com).

### Potentiatior compounds

Potentiator compounds used in the composition of the present invention and in the compositions disclosed herein are commercially available, for example, from Enamine, a chemical supplier company, (www.enamine.com).

The name of compound of formula (I) is *N*-[2-(dimethylamino)ethyl]-3-[(9H-fluoren-2-ylcarbamoylamino)methyl]benzamide.

The structures of the four potentiator compounds defined by general formula (II), including the IUPAC name, are shown in Table 1:

**TABLE 1**

| **Compound** | **Chemical structure and IUPAC name** |
|---|---|
| (IIA) | 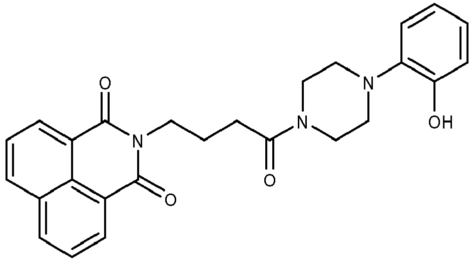 |
| | 2-[4-[4-(2-hydroxyphenyl)piperazin-1-yl]-4-oxobutyl]benzo[de]isoquinoline-1,3-dione |
| (IIB) | 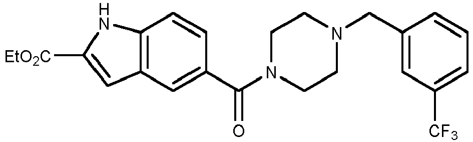 |
| | ethyl 5-[4-[[3-(trifluoromethyl)phenyl]methyl]piperazine-1-carbonyl]-1 *H*-indole-2-carboxylate |
| (IIC) | 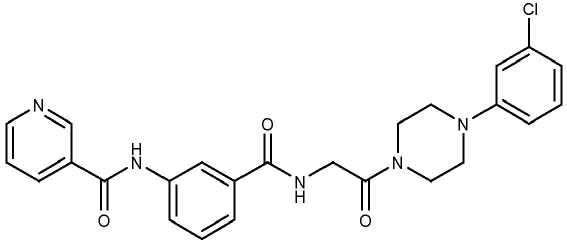 |
| | *N*-[3-[[2-[4-(3-chlorophenyl)piperazin-1-yl]-2-oxoethyl]carbamoyl]-phenyl]pyridine-3-carboxamide |
| (IID) | 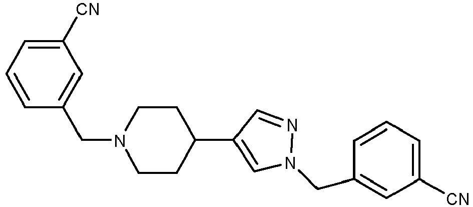 |
| | 3-[[4-[1-[(3-cyanophenyl)methyl]pyrazol-4-yl]piperidin-1-yl]methyl]-benzonitrile |

### Composition

The composition of the invention comprises a therapeutically effective amount of an antibiotic.

The antibiotic is selected from the group disclosed above. In a preferred embodiment, the antibiotic is selected from aminoglycosides like, for example, amikacin, gentamicin, or tobramycin; carbapenems like, for example, imipenem or meropenem; cephalosporins like, for example, ceftazidime, cefepime, or ceftriaxone; penicillins like, for example, piperacillin, optionally combined with tazobactam; polymyxins like, for example, colistin, or polymyxin B; quinolones like, for example, levofloxacin; sulphonamides like, for example, sulfamethoxazole, optionally combined with trimethoprim; and tetracyclines like, for example, minocycline, or tigecycline.

In a preferred embodiment the antibiotic is selected from the group consisting of polymyxin antibiotics, ciprofloxacin, amikacin, levofloxacin, tigecycline, imipenem and the combination trimethoprim/sulfamethoxasole, preferably in a relationship of 1:5 or 1:19.

In a more preferred embodiment, the antibiotic is a polymyxin antibiotic. In a more preferred embodiment the polymyxin antibiotic is selected from colistin, colistimethate, and pharmaceutically acceptable salts thereof, in a yet more preferred embodiment the polymyxin antibiotic is selected from colistin, colistin sulfate and colistimethate sodium, in a yet more preferred embodiment is colistin or colistin sulfate.

In a preferred embodiment the potentiator is compound of formula (I). In a more preferred embodiment the potentiator is compound of formula (I) and the antibiotic is selected from the group consisting of polymyxin antibiotics, ciprofloxacin, amikacin, levofloxacin, tigecycline, imipenem and the combination trimethoprim/sulfamethoxasole. In a more preferred embodiment the potentiator is compound of formula (I) and the polymyxin antibiotic is selected from colistin, colistin sulfate and colistimethate sodium. In a yet more preferred embodiment, the potentiator is compound of formula (I) and the polymyxin antibiotic is colistin or colistin sulfate.

The antibiotic composition according to the invention may, for example, take the form of ointments, gels, pastes, creams, sprays (including aerosols), lotions, suspensions, solutions and emulsions of the active ingredient in aqueous or nonaqueous diluents, syrups, granulates or powders.

The diluents to be used in pharmaceutical compositions (e.g. granulates) adapted to be formed into tablets, dragees, capsules and pills include the following: (a) fillers and extenders, e.g. starch, sugars, mannitol, and silicic acid; (b) binding agents, e.g. carboxymethyl cellulose and other cellulose derivatives, alginates, gelatine and polyvinyl pyrrolidone; (c) moisturizing agents, e.g. glycerol; (d) disintegrating agents, e.g. agar-agar, calcium carbonate and sodium bicarbonate; (e) agents for retarding dissolution e.g. paraffin; (f) resorption accelerators, e.g. quaternary ammonium compounds; (g) surface active agents, e.g. cetyl alcohol, glycerol monostearate; (h) adsorptive carriers, e.g. kaolin and bentonite; (i) lubricants, e.g. talc, calcium and magnesium stearate and solid polyethylene glycols.

The tablets, dragees, capsules and pills formed from the pharmaceutical compositions of the invention can have the customary coatings, envelopes and protective matrices. They can be so constituted that they release the active ingredient only or preferably in a particular part of the intestinal tract, possibly over a period of time. The coatings, envelopes and protective matrices may be made, for example, of polymeric substances or waxes.

The ingredient can also be made up in microencapsulated form together with one or several of the above-mentioned diluents.

The diluents to be used in pharmaceutical compositions adapted to be formed into suppositories can, for example, be the usual water-soluble diluents, such as polyethylene glycols and fats (e.g. cocoa oil and high esters (e.g. C₁₄-alcohol with C₁₆-fatty acid)) or mixtures of these diluents.

The pharmaceutical compositions which are ointments, pastes, creams and gels can, for example, contain the usual diluents, e.g. animal and vegetable fats, waxes, paraffins, starch, tragacanth, cellulose derivatives, polyethylene glycols, silicones, bentonites, silicic acid, talc and zinc oxide or mixtures of these substances.

The pharmaceutical compositions, which are powders and sprays can, for example, contain the usual diluents, e.g. lactose, talc, silicic acid, aluminium hydroxide, calcium silicate, and polyamide powder or mixtures of these substances. Aerosol sprays can, for example, contain the usual propellants, e.g. chlorofluorohydrocarbons.

The pharmaceutical compositions which are solutions and emulsions can, for example, contain the customary diluents, such as solvents, dissolving agents and emulsifiers; specific examples of such diluents are water, ethyl alcohol, isopropyl alcohol, ethyl carbonate, ethyl acetate, benzyl alcohol, benzyl benzoate, propylene glycol, 1,3-butylene glycol, dimethylformamide, dimethylsulfoxide, oils (for example ground nut oil), glycerol, tetrahydrofurfuryl alcohol, polyethylene glycols and fatty acid esters of sorbitan or mixtures thereof.

For parenteral administration, solutions and emulsions should be sterile, and, if appropriate, blood-isotonic.

The pharmaceutical compositions which are suspensions can contain the usual diluents, such as liquid diluents, e.g. water, ethyl alcohol, propylene glycol, surface-active agents (e.g. ethoxylated isostearyl alcohols, polyoxyethylene sorbitan and sorbitan esters), microcrystalline cellulose, bentonite, agar-agar and tragacanth or mixtures thereof.

All the pharmaceutical compositions according to the invention can also contain colouring agents and preservatives as well as flavouring additions (e.g. peppermint oil and eucalyptus oil) and sweetening agents (e.g. saccharin).

The composition of the invention may be, for example, for oral, parenteral, nasal administration. In a preferred embodiment, the administration is parenteral, and more preferably it is administered intravenously.

Parenteral administration may be carried out, for example, subcutaneous, intramuscular, intravenous, or intradermal. Parenteral compositions suitable for administering the composition of the invention may be, for example, in the form of solution, emulsion, suspension, or powder for injection.

The pharmaceutically acceptable excipient may be among those disclosed in well-known handbooks of pharmaceutical technology such as, for example, R.C. Rowe et al., Handbook of Pharmaceutical Excipients, 4th ed., Pharmaceutical Press, London, 2003 [ISBN: 0-85369-472-9]. The selection of the excipients depends on the pharmaceutical composition to be prepared. The preparation of pharmaceutical compositions is well-known by the skilled person in the art, and it is disclosed, for example, in the handbook Remington The Science and Practice of Pharmacy, 20th ed., Lippincott Williams & Wilkins, Philadelphia, 2000 [ISBN: 0-683-306472].

In the case of colistin and pharmaceutically acceptable salts thereof, it is usually administered intravenously in a dosage comprised between 0.01 and 6 mg/kg per day, preferably between 0.05 and 4 mg/kg per day, and more preferably between 0.25 and 2 mg/kg per day, expressed as colistin base.

The amount of the potentiator is comprised between 0.1 and 10 mg/kg per day, preferably between 0.5 and 5 mg/kg per day, and more preferably between 1 and 4 mg/kg per day.

By using any of these amounts of potentiator, the antibacterial activity of the antibiotic is enhanced relative to the antibacterial activity of the antibiotic alone.

In the composition of the invention, the antibiotic and the potentiator may be in the same dosage form, or may be in separated dosage forms. Preferably both are in the same dosage form.

The administration of the antibiotic may be simultaneous to the potentiator administration, or the antibiotic may be administered before or after the potentiator. In a preferred embodiment, the antibiotic and the potentiator are administered simultaneously.

It forms also part of the object of the present invention the antibiotic composition, which comprises a therapeutic amount of an antibiotic, one potentiator compound of formula (I) as shown above, and at least one pharmaceutically acceptable excipient, for use as a medicament.

It is also disclosed herein the antibiotic composition, which comprises a therapeutic amount of an antibiotic, one or more potentiator compounds of formula (I) or (II) as shown above, and at least one pharmaceutically acceptable excipient, for use as a medicament.

### Effect of the potentiator compounds

Antibiotic compositions of the present invention, comprising an antibiotic and one potentiator compound of formula (I) and antibiotic compositions disclosed herein comprising an antibiotic and one potentiator compound of formula (II), were tested against Gram-negative collection isolates representing the most common agents of nosocomial infections (*Escherichia coli* ATCC 25922, *E. coli* ATCC 9637, *Salmonella enterica* subsp. *enterica* ATCC 14028, *Acinetobacter baumannii* ATCC 15308, *Pseudomonas aeruginosa* ATCC 27853, *P. aeruginosa* PAO1, *Stenotrophomonas maltophilia* ATCC 13637 and *Enterobacter aerogenes* ATCC 13048) and against resistant clinical isolates of *Stenotrophomonas maltophilia* and *Acinetobacter baumannii.*

The potentiator effect of the five compounds tested is apparent in the susceptibilities of clinical isolates resistant to several antibiotics, such as *Stenotrophomonas maltophilia* and *Acinetobacter baumannii* from diverse clinical sources. Clinical strains of both bacteria are often resistant to multiple antibiotics, although the mechanisms of resistance are generally poorly understood. Results for the microdilution assay, by determining the growth inhibition at different antibiotic concentrations in the presence of 0.1 mM of selected potentiator compounds, revealed significant increase in susceptibilities for most strains tested. In the case of colistin, the microdilution assay revealed significant increase in susceptibilities for all strains tested.

As shown in Examples 1 and 2, the potentiator compounds increase the susceptibility to the antibiotic in at least one of the most common agents of nosocomial infections and at least one of the tested resistant clinical isolates, being compound of formula (I) the best one.

As shown in Examples 3 and 4, compositions of the invention and other compositions disclosed herein are useful in suppressing the growth of two strains of *Stenotrophomonas maltophilia,* which are resistant to the antibiotic colistin in the absence of potentiators.

As shown in Examples 5, the compound with formula (I) expands the spectrum of coverage of the compositions of the invention since it shows bactericidal activity against the Gram-positive bacterium *Staphylococcus aureus.*

As shown in Examples 6, compositions of the invention, where the compound with formula (I) was first solubilized in methanol, are useful in suppressing the growth of two strains of *S*. *maltophilia,* which are resistant to the antibiotic colistin in the absence of potentiators.

Therefore, it forms also part of the object of the invention a compound of general formula (I): 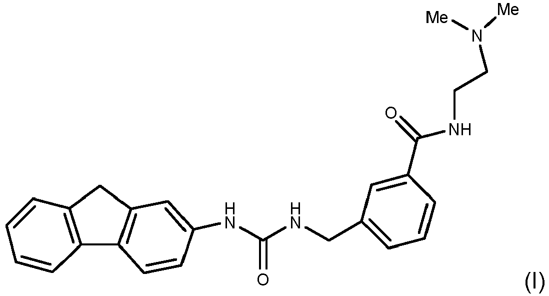 for use in the treatment of bacterial infections as potentiator of the antibacterial activity of an antibiotic.

It is also disclosed herein a compound selected from a group consisting of:
a) a compound of general formula (I): 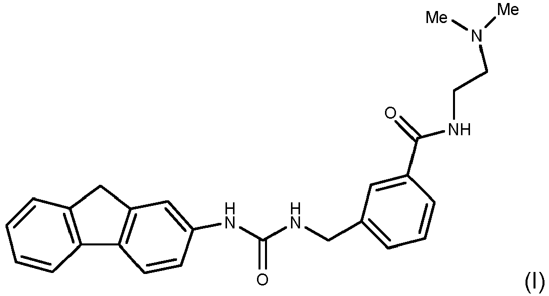 and
b) a compound of formula (II): 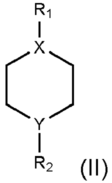 wherein:
   when X = Y = N (nitrogen atom)
   R₁ is 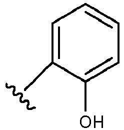
   and R₂ is 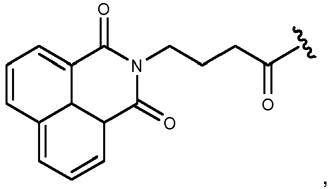
   or R₁ is 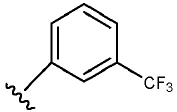
   and R₂ is 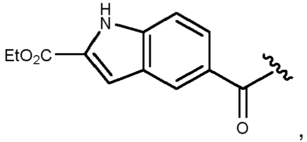
   or R₁ is 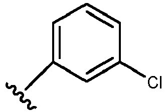
   and R₂ is 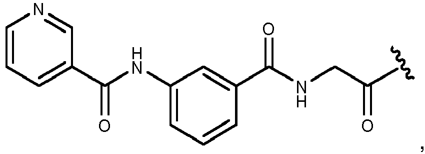 and
   when X = CH and Y = N (nitrogen atom),
   R₁ is 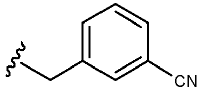
   and R₂ is 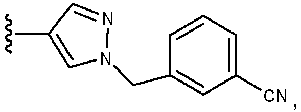 for use as potentiator of the antibacterial activity of an antibiotic.

In a preferred embodiment the antibiotic is selected from aminoglycosides like, for example, amikacin, gentamicin, or tobramycin; carbapenems like, for example, imipenem or meropenem; cephalosporins like, for example, ceftazidime, cefepime, or ceftriaxone; penicillins like, for example, piperacillin, optionally combined with tazobactam; polymyxin like, for example, colistin, or polymyxin B; quinolones like, for example, levofloxacin; sulphonamides like, for example, sulfamethoxazole, optionally combined with trimethoprim; and tetracyclines like, for example, minocycline, or tigecycline. In a more preferred embodiment the antibiotic is selected from the group consisting of polymyxin antibiotic, ciprofloxacin, amikacin, levofloxacin, tigecycline, imipenem and the combination trimethoprim/sulfamethoxasole, preferably in a relationship of 1:5 or 1:19. In a more preferred embodiment, the antibiotic is a polymyxin antibiotic; in a yet more preferred embodiment, the polymyxin antibiotic is selected from colistin, colistin sulfate and colistimethate sodium. In a yet more preferred embodiment, the polymyxin antibiotic is colistin or colistin sulfate.

In a more preferred embodiment the potentiator is compound of formula (I) and the antibiotic is selected from aminoglycosides like, for example, amikacin, gentamicin, or tobramycin; carbapenems like, for example, imipenem or meropenem; cephalosporins like, for example, ceftazidime, cefepime, or ceftriaxone; penicillins like, for example, piperacillin, optionally combined with tazobactam; polymyxin like, for example, colistin, or polymyxin B; quinolones like, for example, levofloxacin; sulphonamides like, for example, sulfamethoxazole, optionally combined with trimethoprim; and tetracyclines like, for example, minocycline, or tigecycline. In a more preferred embodiment the potentiator is compound of formula (I) and the antibiotic is selected from the group consisting of polymyxin antibiotic, ciprofloxacin, amikacin, levofloxacin, tigecycline, imipenem and the combination trimethoprim/sulfamethoxasole, preferably in a relationship of 1:5 or 1:19. In a more preferred embodiment, the potentiator is compound of formula (I) and the antibiotic is a polymyxin antibiotic, in a yet more preferred embodiment, the potentiator is compound of formula (I) and the polymyxin antibiotic is selected from colistin, colistin sulfate and colistimethate sodium. In a yet more preferred embodiment, the potentiator is compound of formula (I) and the polymyxin antibiotic is colistin or colistin sulfate.

Surprisingly, the compounds of formula (I) or (II) enhance the susceptibility to antibiotics, in particular to colistin, being possible to formulate an effective antibiotic composition with a lower dosage of antibiotic and, consequently showing reduced toxicity, or maintaining the dosage of antibiotic and providing an antibiotic composition with improved activity. The best potentiating effect is provided by compound of formula (I).

Next, several examples of the invention are provided for illustrative but not limitative purposes.

### Examples

### Example 1: Enhanced antibacterial composition in front of Gram-negative bacteria

The potentiator effect of the compound of the invention and other compounds disclosed herein was tested using colistin sulfate as antibiotic and in combination with the enhancer compounds in front of specific Gram-negative bacteria, representing the most common agents of nosocomial infections.

For this experiment the gram-negative reference isolates shown in Table 2 were used:

**TABLE 2**

| **Species and strain designation** | **Spanish Type Culture Collection** (CECT) |
|---|---|
| *Escherichia coli* ATCC 25922 | CECT 434 |
| *Escherichia coli* ATCC 9637 | CECT 4099 |
| *Salmonella enterica* subsp. *enterica* ATCC 14028 | CECT 4594 |
| *Acinetobacter baumannii* ATCC 15308 | CECT 452 |
| *Pseudomonas aeruginosa* ATCC 27853 | CECT 108 |
| *Pseudomonas aeruginosa* PAO1 | - |
| *Stenotrophomonas maltophilia* ATCC 13637 | CECT 115 |
| *Enterobacter aerogenes* ATCC 13048 | CECT 684 |

Reference strains, except *P. aeruginosa* PAO1, were obtained from the Spanish Type Culture Collection (CECT) in Valencia, CECT reference numbers are indicated in Table 1. *P. aeruginosa* strain PAO1 was obtained from the two-allele transposon mutant library at the University of Washington (Seattle, WA) (Jacobs, M. A. et al. Comprehensive transposon mutant library of Pseudomonas aeruginosa, Proc. Natl. Acad. Sci. U. S. A., 2003, 100, 14339-14344).

All the isolates were stored at -80° C until further testing.

Minimal inhibitory concentration (MIC) of the antibiotic or the test combinations were determined by the broth microdilution method.

Antimicrobial susceptibility testing of these isolates was performed using the broth microdilution (BMD) method, according to CLSI (formerly National Committee on Clinical Laboratory Standards, NCCLS) guidelines (Clinical and Laboratory Standards Institute (CLSI). Methods for dilution antimicrobial susceptibility tests for bacteria that grow aerobically; approved standard. 9th ed., M07-A9. Wayne, PA, USA, 2012, 32).

The Mueller-Hinton broth (MHB) was purchased from OXOID (Oxoid Ltd, Basingstoke, Hampshire, UK; Product number CMO405, Lot 1460487) and was prepared from the powder and supplemented with Ca²⁺ and Mg²⁺ to obtain cation-adjusted MHB (CAMHB) according to CLSI guidelines. For Ca²⁺, the final amount needed was 25 mg/L and for Mg²⁺, the final amount needed was 12.5 mg/L.

Colistin stock solutions were prepared by dissolving colistin sulfate powder (Apollo Scientific Ltd, BatchAS405305) in Milli-Q water and sterilizing by passage through 0.20 µm filters.

BMD with MHB was performed in accordance with the CLSI recommendations and was used as the reference method. Briefly, serial twofold dilutions of the test antibiotic were made in 100 µL of MHB using sterile 96-well polystyrene microtitre plates (BrandTech 781662).

Colistin sulfate concentrations ranging from 0.0125 µg/mL to 512 µg/mL were tested by the BMD method in combination with the compound of the invention and other compounds disclosed herein. Selected enhancer compounds were added to each antibiotic dilution to a fixed final concentration of 0.1 mM.

As controls, the antibiotic was tested alone in the assay broth or in the presence of 1% DMSO.

Selected compounds were first dissolved in 100% of sterile-filtered dimethyl sulfoxide (DMSO, Sigma, D2438) to 10 mM each. 100 µL of bacterial suspension (final OD₅₅₀ₙₘ=0.01) were added to the antibiotic dilutions in each well, and the final suspension was mixed and incubated without shaking at 37° C for 24 h before developing with 30 µL of a 0.01% aqueous solution of resazurin (Nateche, F. et al., Application of the resazurin microtitre assay for detection of multidrug resistance in Mycobacterium tuberculosis in Algiers, J. Med. Microbiol., 2006, 55, 857-860).

For the experiment all bacteria were first grown overnight in CAMHB using CLSI recommended incubation conditions.

The MIC was defined as the lowest concentration of colistin at which no visible growth was obtained. Susceptibility fold increase was expressed for each isolate as the MIC for colistin divided by the MIC for colistin in the presence of the enhancer compound.

Previously to the potentiator assay, the MIC breakpoints to colistin for all the reference bacterial isolates were determined. The reference bacteria were classified as resistant or susceptible to colistin according to the CLSI criteria. The MIC for colistin for all these reference strains ranged from 0.5 to >4.0.

CLSI's statements for the susceptibility testing of colistin as disclosed in Clinical and Laboratory Standards Institute (CLSI). Performance standards for antimicrobial susceptibility testing: twenty-second informational supplement M100-S22. Wayne, PA, USA. 2012, 32, are:
1) breakpoints for *P. aeruginosa* are: susceptibility, MIC ≤2 mg/L; intermediate, MIC = 4 mg/L; and resistance, MIC ≥8 mg/L;
2) breakpoints for *Acinetobacter spp.* are: susceptibility, MIC <4 mg/L; resistance, MIC ≥4 mg/L.

CLSI recommendations for *S*. *maltophilia, Enterobacteriaceae* and many other bacteria do not exist currently.

Colistin minimal inhibitory concentration (MIC), expressed in µg/mL, and the fold increase in bacterial susceptibility to colistin when combined with the potentiator compound (expressed as MIC in the presence of 1.0% DMSO / MIC in the presence of 0.1 mM of each potentiator compound and 1.0% DMSO) are shown in Table 3:

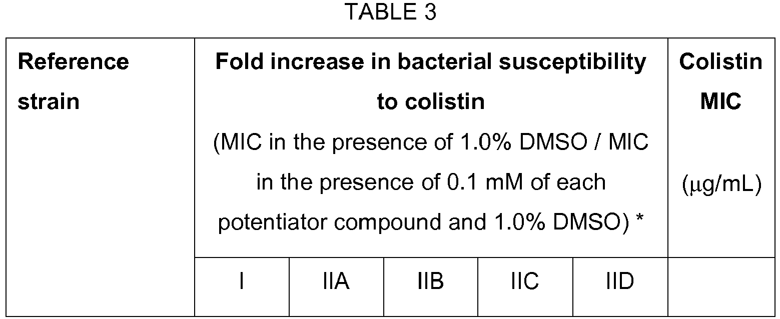

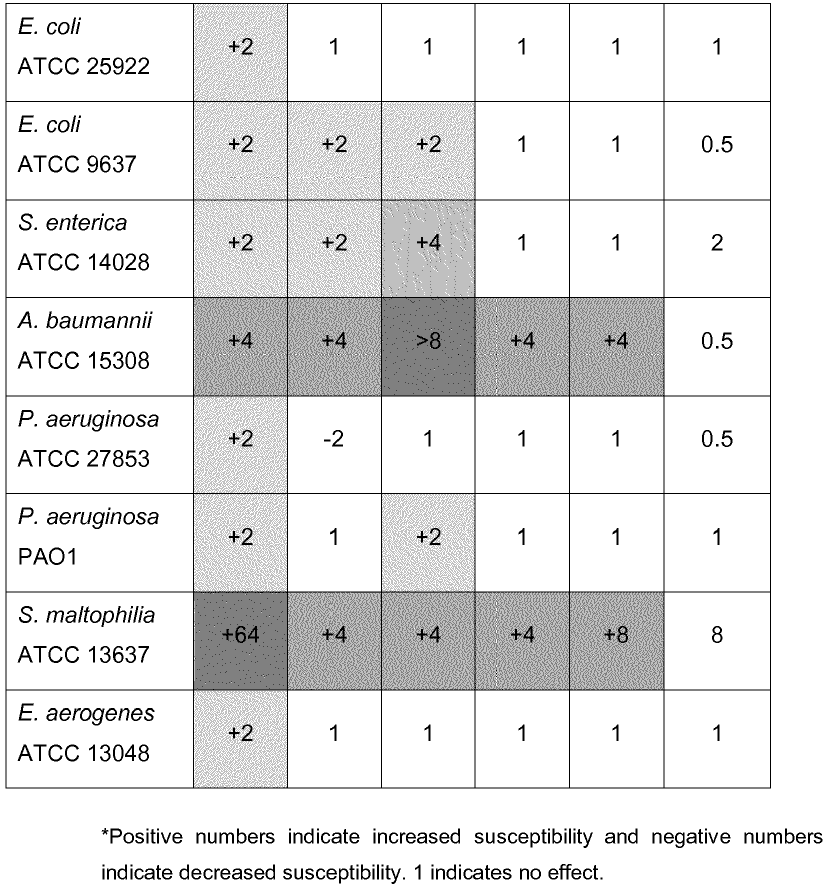

It can be observed that the potentiator compounds increase the susceptibility to colistin in at least one of the most common agents of nosocomial infections. Compound of formula I showed the best potentiator activity.

### Example 2: Enhanced antibacterial composition in front of Gram-negative bacteria

Following the procedure of Example 1, ciprofloxacin, amikacin, levofloxacin, tigecycline, imipenem and the combination trimethoprim/sulfamethoxasole (1:19) were tested. All were purchased from Sigma-Aldrich Co.

Minimal inhibitory concentration (MIC) of the antibiotic or the test combinations were determined by the broth microdilution method.

Antibiotic stock solutions were prepared according to CLSI recommendations and sterilizing by passage through 0.20 µm filters.

Antimicrobial susceptibility testing of these isolates was performed using the broth microdilution (BMD) method, according to CLSI guidelines.

The fold increase in bacterial susceptibility to antibiotics when combined with the potentiator compound (expressed as MIC in the presence of 1.0% DMSO / MIC in the presence of 0.1 mM of each potentiator compound and 1.0% DMSO) are shown in Table 4:

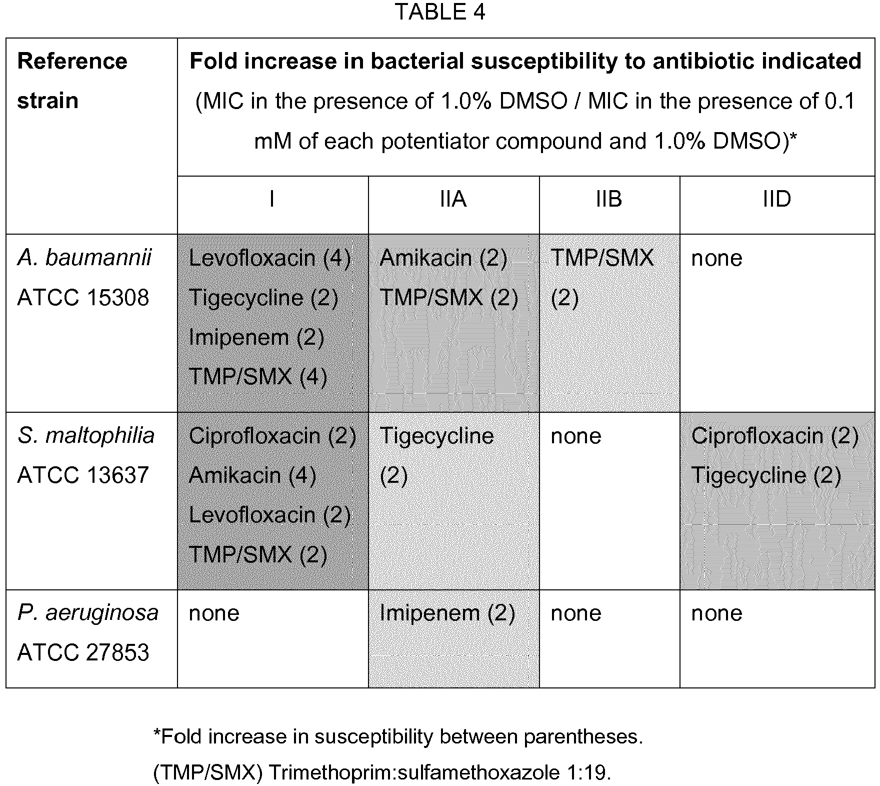

It can be observed that the potentiator compounds increase the susceptibility to different types of antibiotics in at least one of the most common agents of nosocomial infections. Compound of formula I showed the best potentiator activity.

### Example 3: Enhanced antibacterial composition in front of resistant clinical isolates

The potentiator effect of the compound of the invention and other compounds disclosed herein was tested using colistin sulfate as antibiotic and in combination with the enhancer compounds in front of resistant clinical isolates.

Tests were performed to determine the potentiator effect of the compound of the invention and other compounds disclosed herein with multidrug-resistant S. *maltophilia* and *A. baumannii* clinical strains as shown in Table 5:

**TABLE 5**

| **Species** | **Isolate designation** | **Isolated from source** |
|---|---|---|
| *Stenotrophomonas maltophilia* | M30 | decubitus ulcer |
| *Stenotrophomonas maltophilia* | 257 | perineum swab |
| *Stenotrophomonas maltophilia* | 197 | oropharynx swab |
| *Stenotrophomonas maltophilia* | 53149 | infected surgical wound |
| *Stenotrophomonas maltophilia* | D457¹ | model of multiresistance |
| *Acinetobacter baumannii* | 6963 | blood sample |
| *Acinetobacter baumannii* | 6799 | aspiration sample |

| | | |
|---|---|---|
| ¹ *S*. *maltophilia* strain D457 is being used as a model for studying antibiotic resistance in this bacterial species since it easily generates spontaneous single-step multiresistance mutant. | | |

The origin, propagation and conservation of these isolates has been described previously (Huedo et al., Two Different rpf Clusters Distributed among a Population of Stenotrophomonas maltophilia Clinical Strains Display Differential Diffusible Signal Factor Production and Virulence Regulation, J. Bacteriol., 2014, 196, 2431-2442, and Ferrer-Navarro et al., Abundance of the Quorum-Sensing Factor Ax21 in Four Strains of Stenotrophomonas maltophilia Correlates with Mortality Rate in a New Zebrafish Model of Infection, PloS One, 2013, 8, e67207).

For this experiment the potentiator compound was added to a colistin broth microdilution assay at a final concentration of 0.1mM. MICs were determined as described in the Example 1.

Colistin minimal inhibitory concentration (MIC), expressed in µg/mL, and the fold increase in bacterial susceptibility to colistin when combined with the potentiator compound (expressed as MIC in the presence of 1.0% DMSO / MIC in the presence of 0.1 mM of each potentiator compound and 1.0% DMSO) are shown in Table 6:

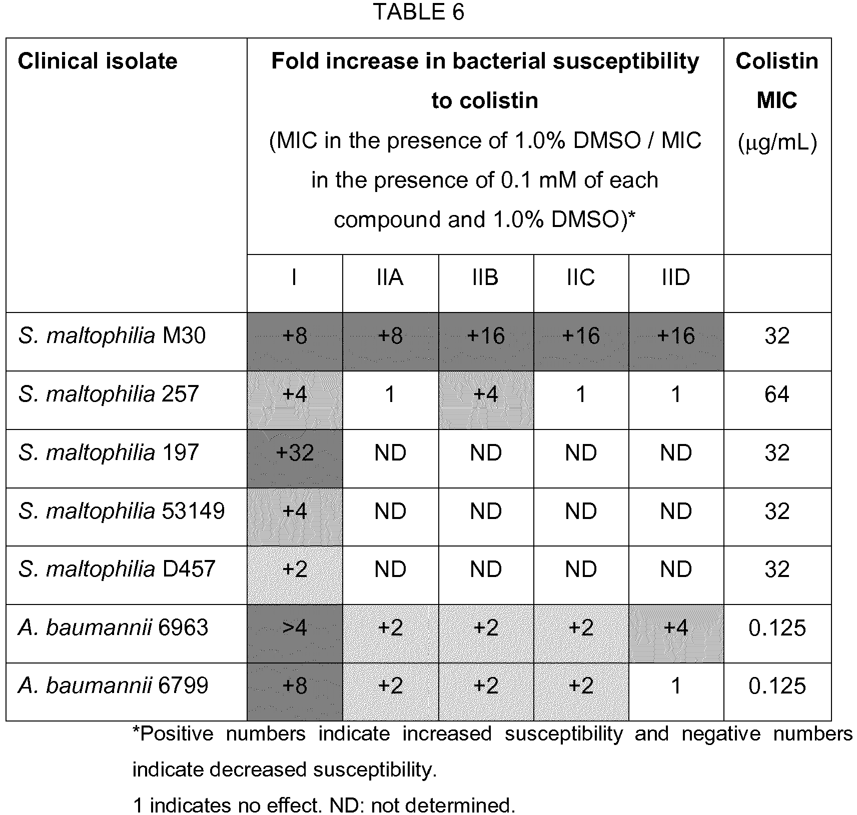

It can be observed that the potentiator compounds increase the susceptibility to colistin in at least one of the resistant clinical isolates. The enhancer capacity was higher for compound of formula I and in the presence of this compound, two strains (M30 and 197) become sensitive to colistin according to the CLSI criteria (Clinical and Laboratory Standards Institute (CLSI). Performance standards for antimicrobial susceptibility testing: twenty-second informational supplement M100-S22. Wayne, PA, USA. 2012, 32).

### Example 4: Enhanced antibacterial composition in front of resistant clinical isolates

Following the procedure of Example 3, ciprofloxacin, amikacin, levofloxacin, imipenem and the combination trimethoprim/sulfamethoxasole (1:19) were tested. All were purchased from Sigma-Aldrich Co.

Minimal inhibitory concentration (MIC) of the antibiotic or the test combinations were determined by the broth microdilution method.

Antibiotic stock solutions were prepared according to CLSI recommendations and sterilizing by passage through 0.20 µm filters.

Antimicrobial susceptibility testing of these isolates was performed using the broth microdilution (BMD) method, according to CLSI guidelines.

The fold increase in bacterial susceptibility to antibiotics when combined with the potentiator compound (expressed as MIC in the presence of 1.0% DMSO / MIC in the presence of 0.1 mM of each potentiator compound and 1.0% DMSO) are shown in Table 7:

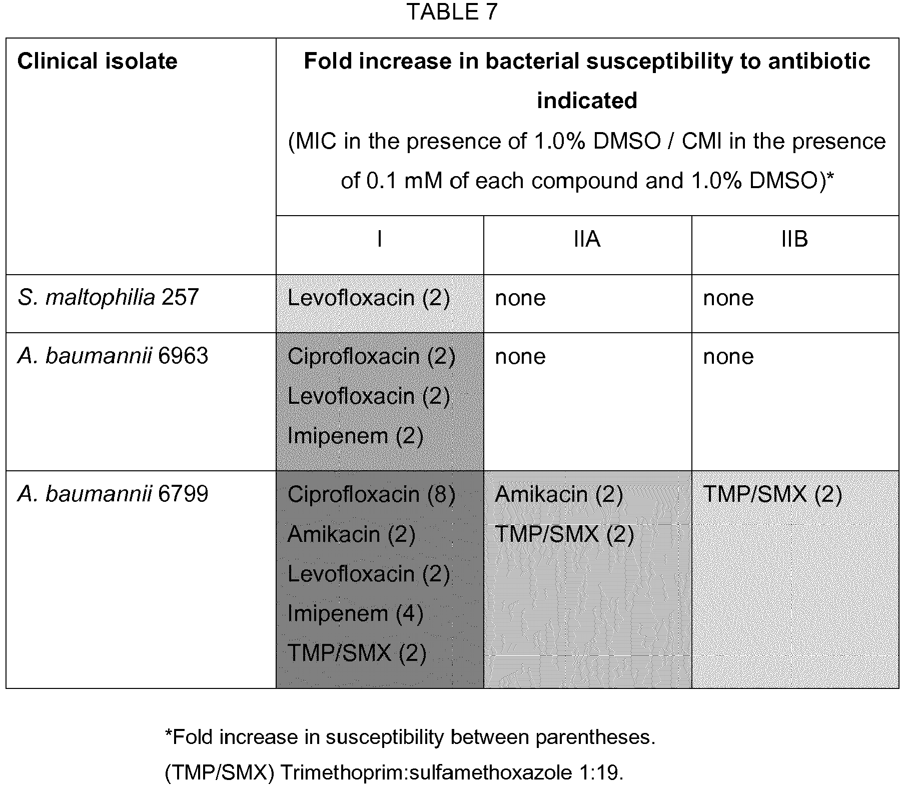

It can be observed that the potentiator compounds increase the susceptibility to different antibiotics in at least one of the resistant clinical isolates. The enhancer capacity was higher for compound of formula I.

### Example 5: Antibacterial composition in front of a Gram-positive bacteria

The antimicrobial effect of the compound of formula I of the invention was tested in front of specific Gram-positive bacterium, *Staphylococcus aureus,* an agent usually involved in hospital-acquired infections. For this experiment the S. *aureus* reference and clinical isolates shown in Table 8 were used, including Methicillin-resistant S. *aureus* (MRSA):

**TABLE 8**

| Species and strain designation | Source (relevant characteristics) | Compound of formula I MIC in the presence of 1.0% DMSO |
|---|---|---|
| *S. aureus* F-182 | Reference strain, ATCC 43300 (CLSI standard) | 0.2 mM |
| *S. aureus* Seatle 1945 | Reference strain, ATCC 25923 | 0.2 mM |
| *S. aureus* V8 | Reference strain, ATCC 49775 | 0.2 mM |
| *S. aureus* Newman | Reference strain, NBRC 102137 | 0.2 mM |
| *S. aureus* USA 300 | Reference strain, ATCC BAA-1556 (MRSA) | 0.2 mM |
| *S. aureus* RN4220 | Reference strain, RN4220 (laboratory strain used in virulence research) | 0.2 mM |
| *S. aureus* RN6390 | Reference strain, RN6390 (laboratory virulent strain). | 0.2 mM |
| *S. aureus* RN6911 | Reference strain, RN6911 (RN6390 with an agr mutation) | 0.2 mM |
| *S. aureus* SA0001 | Clinical strain, (resistant to penicillin) | 0.2 mM |
| *S. aureus* SA0002 | Clinical strain, (resistant to penicillin) | 0.1 mM |
| *S. aureus* SA0003 | Clinical strain, (resistant to penicillin) | 0.1 mM |
| *S. aureus* SA0004 | Clinical strain, (MRSA, and resistant to ciprofloxacin and cloxacillin) | 0.2 mM |

| | | |
|---|---|---|
| Note: ATCC, American Type Culture Collection; NBRC, NITE Biological Resource Center (Japan). | | |

All clinical strains were obtained either from upper respiratory tract colonization or infection or from bacteraemia. All the isolates were stored at -80° C until further testing.

Antimicrobial susceptibility testing of these isolates was performed using the broth microdilution (BMD) method, according to CLSI guidelines (Example 1).

Selected compound of formula I was first dissolved in 100% of sterile-filtered dimethyl sulfoxide (DMSO, Sigma, D2438) to 20 mM. 100 µL of bacterial suspension (final OD550nm=0.01) in MHB were added to the compound dilutions in each well (concentration range from 0.2 mM to 3.125 µM), and the final suspension was mixed and incubated without shaking at 37° C for 24 h before developing with 30 µL of a 0.01% aqueous solution of resazurin. For the experiment all bacteria were first grown overnight in CAMHB using CLSI recommended incubation conditions.

The compound MIC, expressed in mM, was defined as the lowest concentration of compound at which no visible growth was obtained.

It can be observed in Table 8 that the compound of formula I has antibacterial activity antibacterial activity against the Gram-positive bacterium S. *aureus* with MIC values ranging between 0.2 to 0.1 mM.

### Example 6: Enhanced antibacterial composition containing compound of formula (I) prepared in Methanol

The potentiator effect of the compound of the invention in a new formulation was tested by using colistin sulphate as antibiotic in combination with the enhancer compound of formula I in front of resistant clinical isolates.

In this example the compound of formula I of the invention was evaluated by preparing the compound stock solution in Methanol instead of in dimethyl sulfoxide (DMSO).

Tests were performed to determine the potentiator effect of the compound of the invention with two colistin resistant *S. maltophilia* strains as shown in Table 9. *S. maltophilia* K279a is a reference clinical strain. The origin of clinical strain OC323 together with K279a has been described previously (Huedo et al., Two Different rpf Clusters Distributed among a Population of Stenotrophomonas maltophilia Clinical Strains Display Differential Diffusible Signal Factor Production and Virulence Regulation, J. Bacteriol., 2014, 196, 2431-2442).

Selected compound of formula I was first dissolved in 100% of Methanol (HPLC Gradient Grade CHROMASOLV. Sigma, 34885-1L-R) to 10 mM. The compound was fairly soluble in methanol up to 10 mM and required vigorous vortex.

For this experiment the potentiator compound was added to a colistin broth microdilution assay at a final concentration of 0.1mM (final methanol concentration of 1%). MICs were determined as described in the Example 1.

Strains colistin minimal inhibitory concentration (MIC), expressed in µg/mL, and the fold increase in bacterial susceptibility to colistin when combined with the potentiator compound previously dissolved in methanol (expressed as MIC in the presence of 1.0% Methanol / MIC in the presence of 0.1 mM of each potentiator compound and 1.0% Methanol) are shown in Table 9:

**TABLE 9**

| Resistant strains (MIC to colistin)* | Fold increase in bacterial susceptibility to colistin | |
|---|---|---|
| | (MIC in the presence of 1.0% Methanol / MIC in the presence of 0.1 mM of compound of formula I and 1.0% Methanol)** | (MIC in the presence of 1.0% DMSO / MIC in the presence of 0.1 mM of compound of formula I and 1.0% DMSO)* |
| *S. maltophilia* K279a (16 µg/mL) | +4 | +4 |
| *S. maltophilia* OC323 (32 µg/mL) | +4 | +4 |

| | | |
|---|---|---|
| * As determined by microdilution test with MHB **Positive numbers indicate increased susceptibility | | |

It can be observed that the potentiator compound of formula I, previously solubilized in methanol, increases the susceptibility to colistin (4-fold) in both resistant strains just as well when it is dissolved in DMSO as in previous examples.

## Claims

1. Antibiotic composition, **characterized in that** it comprises a therapeutic amount of an antibiotic, one potentiator compound which is a compound of general formula (I): 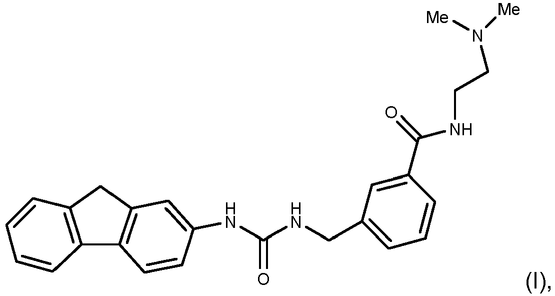 and at least one pharmaceutically acceptable excipient.

2. Antibiotic composition according to claim 1, **characterized in that** the antibiotic is selected from aminoglycosides, carbapenems, cephalosporins, macrolides, monobactams, penicillins, polymyxins, quinolones, fluoroquinolones, sulphonamides, tetracyclines, chloramphenicol and fosfomycin.

3. Antibiotic composition according to claim 2, **characterized in that** the antibiotic is selected from the group consisting of polymyxin antibiotics, ciprofloxacin, amikacin, levofloxacin, tigecycline, imipenem and the combination trimethoprim/sulfamethoxasole.

4. Antibiotic composition according to claim 3, **characterized in that** the antibiotic is a polymyxin antibiotic.

5. Antibiotic composition according to claim 4, **characterized in that** the polymyxin antibiotic is selected from colistin, colistimethate, and pharmaceutically acceptable salts thereof.

6. Antibiotic composition according to claim 5, **characterized in that** the polymyxin antibiotic is selected from colistin, colistin sulfate and colistimethate sodium.

7. Antibiotic composition according to claim 6, **characterized in that** the polymyxin antibiotic is colistin or colistin sulfate.

8. Antibiotic composition of any of claims 1 to 7 for use as a medicament.

9. Antibiotic composition for use according to claim 8, **characterized in that** colistin and pharmaceutically acceptable salts thereof is administered intravenously in a dosage comprised between 0.01 and 6 mg/kg per day expressed as colistin base.

10. Antibiotic composition for use according to claim 8, **characterized in that** the antibiotic and the potentiator are in the same dosage form.

11. A compound of general formula (I): 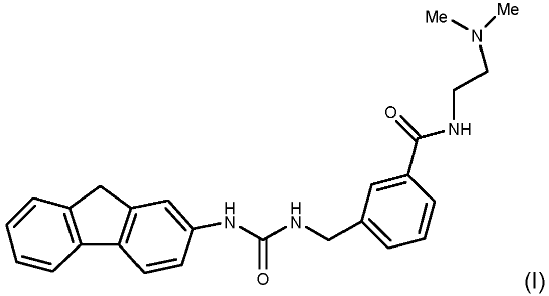 for use in the treatment of bacterial infections as potentiator of the antibacterial activity of an antibiotic.

12. Compound for use according to claim 11, **characterized in that** the antibiotic is selected from the group consisting of polymyxin antibiotics, ciprofloxacin, amikacin, levofloxacin, tigecycline, imipenem and the combination trimethoprim/sulfamethoxasole.

13. Compound for use according to claim 12, **characterized in that** the antibiotic is a polymyxin antibiotic.

14. Compound for use according to claim 13, **characterized in that** the polymyxin antibiotic is selected from colistin, colistin sulfate and colistimethate sodium.

15. Compound for use according to claim 14, **characterized in that** the polymyxin antibiotic is colistin or colistin sulfate.

## Patentansprüche

1. Antibiotika-Zusammensetzung, **dadurch gekennzeichnet, dass** sie eine therapeutische Menge eines Antibiotikums umfasst, eine Potentiatorverbindung, die eine Verbindung der allgemeinen Formel ist (I): 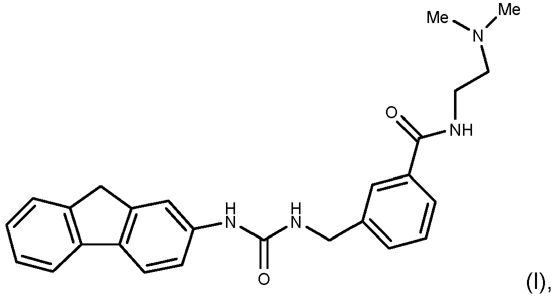 und mindestens einen pharmazeutisch verträglichen Hilfsstoff.

2. Antibiotika-Zusammensetzung nach Anspruch 1, **dadurch gekennzeichnet, dass** das Antibiotikum ausgewählt ist aus Aminoglycosiden, Carbapenemen, Cephalosporinen, Makroliden, Monobactamen, Penicillinen, Polymyxinen, Chinolonen, Fluorchinolonen, Sulfonamiden, Tetracyclinen, Chloramphenicol und Fosfomycin.

3. Antibiotika-Zusammensetzung nach Anspruch 2, **dadurch gekennzeichnet, dass** das Antibiotikum ausgewählt ist aus der Gruppe bestehend aus Polymyxin-Antibiotika, Ciprofloxacin, Amikacin, Levofloxacin, Tigecyclin, Imipenem und der Kombination Trimethoprim / Sulfamethoxasol.

4. Antibiotika-Zusammensetzung nach Anspruch 3, **dadurch gekennzeichnet, dass** das Antibiotikum ein Polymyxin-Antibiotikum ist.

5. Antibiotika-Zusammensetzung nach Anspruch 4, **dadurch gekennzeichnet, dass** das Polymyxin-Antibiotikum ausgewählt ist aus Colistin, Colistimethat und pharmazeutisch verträglichen Salzen davon.

6. Antibiotika-Zusammensetzung nach Anspruch 5, **dadurch gekennzeichnet, dass** das Polymyxin-Antibiotikum ausgewählt ist aus Colistin, Colistinsulfat und Colistimethat-Natrium.

7. Antibiotika-Zusammensetzung nach Anspruch 6, **dadurch gekennzeichnet, dass** das Polymyxin-Antibiotikum Colistin oder Colistinsulfat ist.

8. Antibiotika-Zusammensetzung nach einem der Ansprüche 1 bis 7 zur Verwendung als Medikament.

9. Antibiotika-Zusammensetzung zur Verwendung nach Anspruch 8, **gekennzeichnet dadurch, dass** Colistin und pharmazeutisch verträgliche Salze davon intravenös in einer Dosierung zwischen 0,01 und 6 mg / kg pro Tag, ausgedrückt als Colistin-Base, verabreicht werden.

10. Antibiotika-Zusammensetzung zur Verwendung nach Anspruch 8, **dadurch gekennzeichnet, dass** das Antibiotikum und der Potentiator in der gleichen Dosierungsform vorliegen.

11. Eine Verbindung der allgemeinen Formel (I): 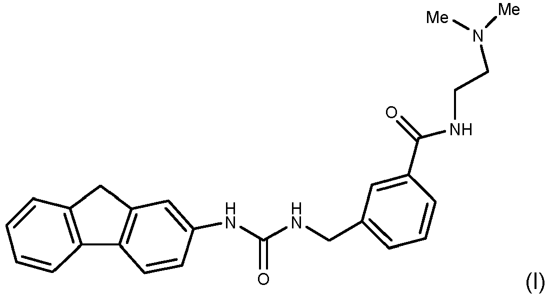 zur Verwendung bei der Behandlung von bakteriellen Infektionen als Potentiator der antibakteriellen Aktivität eines Antibiotikums.

12. Verbindung zur Verwendung nach Anspruch 11, **dadurch gekennzeichnet, dass** das Antibiotikum ausgewählt ist aus der Gruppe bestehend aus Polymyxin-Antibiotika, Ciprofloxacin, Amikacin, Levofloxacin, Tigecyclin, Imipenem und der Kombination Trimethoprim / Sulfamethoxasol.

13. Verbindung zur Verwendung nach Anspruch 12, **dadurch gekennzeichnet, dass** das Antibiotikum ein Polymyxin-Antibiotikum ist.

14. Verbindung zur Verwendung nach Anspruch 13, **dadurch gekennzeichnet, dass** das Polymyxin-Antibiotikum ausgewählt ist aus Colistin, Colistinsulfat und Colistimethat-Natrium.

15. Verbindung zur Verwendung nach Anspruch 14, **dadurch gekennzeichnet, dass** das Polymyxin-Antibiotikum Colistin oder Colistinsulfat ist.

## Revendications

1. Composition antibiotique, **caractérisée en ce qu'**elle comprend une quantité thérapeutique d'un antibiotique, un composé potentialisateur qui est un composé de formule générale (I): 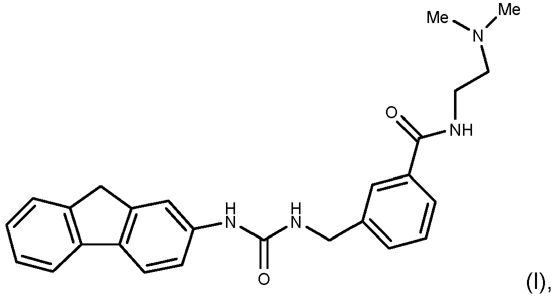 et au moins un excipient pharmaceutiquement acceptable.

2. Composition antibiotique selon la revendication 1, **caractérisée en ce que** l'antibiotique est choisi parmi les aminoglycosides, les carbapénèmes, les céphalosporines, les macrolides, les monobactames, les pénicillines, les polymyxines, les quinolones, les fluoroquinolones, les sulfonamides, les tétracyclines, le chloramphénicol et la fosfomycine.

3. Composition antibiotique selon la revendication 2, **caractérisée en ce que** l'antibiotique est choisi dans le groupe constitué par les antibiotiques polymyxines, la ciprofloxacine, l'amikacine, la lévofloxacine, la tigécycline, l'imipénème et l'association triméthoprime / sulfaméthoxasole.

4. Composition antibiotique selon la revendication 3, **caractérisée en ce que** l'antibiotique est un antibiotique polymyxine.

5. Composition antibiotique selon la revendication 4, **caractérisée en ce que** l'antibiotique polymyxine est choisi parmi la colistine, le colistiméthate et leurs sels pharmaceutiquement acceptables.

6. Composition antibiotique selon la revendication 5, **caractérisée en ce que** l'antibiotique polymyxine est choisi parmi la colistine, le sulfate de colistine et le colistiméthate de sodium.

7. Composition antibiotique selon la revendication 6, **caractérisée en ce que** l'antibiotique polymyxine est la colistine ou le sulfate de colistine.

8. Composition antibiotique selon l'une quelconque des revendications 1 à 7 à utiliser comme médicament.

9. Composition antibiotique à utiliser selon la revendication 8, **caractérisée en ce que** la colistine et ses sels pharmaceutiquement acceptables sont administrés par voie intraveineuse à une posologie comprise entre 0,01 et 6 mg / kg par jour exprimée en colistine base.

10. Composition antibiotique à utiliser selon la revendication 8, **caractérisée en ce que** l'antibiotique et le potentialisateur sont dans la même forme posologique.

11. Un composé de formule générale (I): 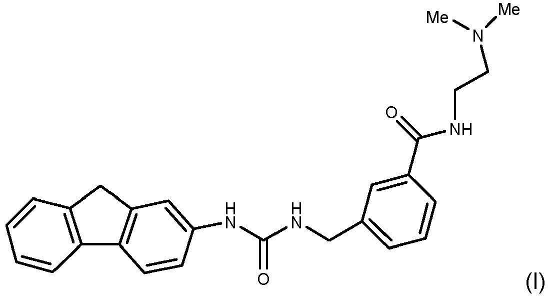 destiné à être utilisé dans le traitement des infections bactériennes en tant que potentialisateur de l'activité antibactérienne d'un antibiotique.

12. Composé à utiliser selon la revendication 11, **caractérisé en ce que** l'antibiotique est choisi dans le groupe constitué par les antibiotiques polymyxines, la ciprofloxacine, l'amikacine, la lévofloxacine, la tigécycline, l'imipénème et l'association triméthoprime / sulfaméthoxasole.

13. Composé à utiliser selon la revendication 12, **caractérisé en ce que** l'antibiotique est un antibiotique polymyxine.

14. Composé à utiliser selon la revendication 13, **caractérisé en ce que** l'antibiotique polymyxine est choisi parmi la colistine, le sulfate de colistine et le colistiméthate de sodium.

15. Composé à utiliser selon la revendication 14, **caractérisé en ce que** l'antibiotique polymyxine est la colistine ou le sulfate de colistine.
